# EUROPEAN PATENT APPLICATION

(11) **EP 2 168 961 A1**
(43) Date of publication of application: **31.03.2010**
(21) Application number: 08778055.7
(22) Date of filing: 04.07.2008
(51) Int. Cl.: C07D 407/06, C07D 295/18, C07F 7/22

(54) **PROCESS FOR PRODUCING MACROLIDE COMPOUND AND INTERMEDIATE THEREFOR**

(30) Priority: 06.07.2007 JP 2007178152
(71) Applicant: Eisai R&D Management Co., Ltd., Tokyo 112-8088 (JP); MERCIAN CORPORATION, Chuo-ku Tokyo 104-8305 (JP)
(72) Inventor: TSUCHIDA, Toshio, Iwata-shi Shizuoka 438-0077 (JP); YOSHIDA, Masashi, Tokyo 166-0003 (JP); OHTA, Kazuo, Iwata-shi Shizuoka 438-0077 (JP); KANEKO, Katsura, Iwata-shi Shizuoka 438-0078 (JP); KOMINATO, Kaichiro, Iwata-shi Shizuoka 438-0078 (JP)
(74) Representative: HOFFMANN EITLE
(86) International application number: PCT/JP2008/062534
(87) International publication number: WO 2009/008492

(57) **Abstract**

The present invention provides a method for producing a 12-membered ring macrolide compound expected as a prophylactic or therapeutic agent for solid tumors and the like, and a production intermediate thereof. In detail, by acetalizing hydroxyl groups at 6- and 7-positions of a 12-membered ring macrolide compound being a raw material with dialkyl tin (IV) oxide and, after that, reacting the product with a carbamoyl halide derivative, the 7-position urethane derivative of the 12-membered ring macrolide compound being the target is effectively produced, without protecting hydroxyl groups at other positions.

## Description

### Technical Field

The present invention relates to a method for producing a 12-membered ring macrolide compound useful as a medicine and a production intermediate thereof.

### Background Art

It is known that a 12-membered ring macrolide compound and derivatives thereof represented by Formula (1) described below have an excellent VEGF (Vascular Endothelial Growth Factor) production-suppressing effect, and that, in addition, they strongly suppress the growth of solid tumor cells in vivo experiments. It is known that a 7-position urethane derivative thereof shows a particularly excellent activity and is expected as a prophylactic or therapeutic agent for solid tumors and the like (refer to WO-A 2003/099813). 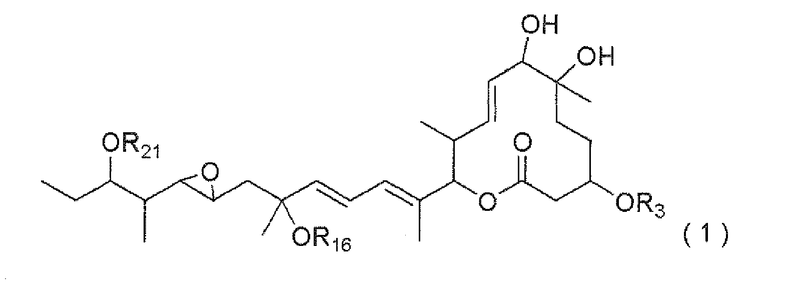 (Where each of R₃, R₁₆ and R₂₁ independently represents a hydrogen atom or a protective group of hydroxyl group.)

As a method for producing the 7-position urethane derivative, the method described in WO-A 2003/099813 is known. That is, the derivative is produced through a process of multiple steps such that, after protecting hydroxyl groups at 3-, 6-, 16- and 21-position of the 12-membered ring macrolide compound 11107D represented by Formula (A) below, which is a raw material, through the use of an appropriate protective group, a 7-position acetyl group is hydrolyzed and removed, and that the product is then treated with a chloroformate derivative or the like in the presence of base to induce a carbonate ester group and, after that, the product is reacted with an intended amine followed by removal of the protective group. Therefore, the development of an effective method having less steps and good yield is required.

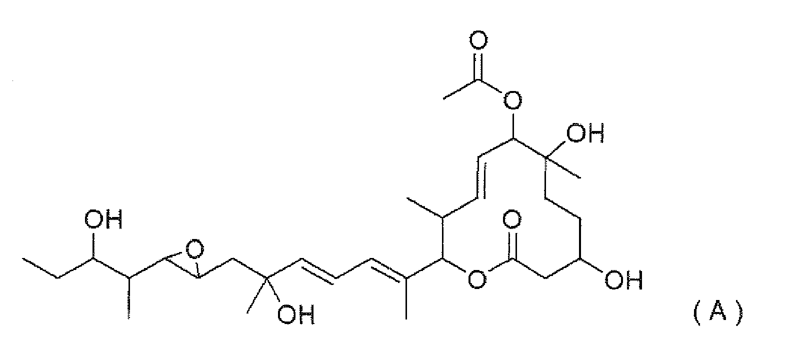

On the other hand, an example is known in the synthesis of acylated derivatives of a 16-membered ring macrolide compound, in which a diol group of a sugar residue of a 16-membered ring macrolide compound is acetalized by using a dialkyl tin compound and is then acylated (see JP-A 62-234093). However, there are no such examples as acetalizing lactone ring diol groups of 12-membered ring macrolide compounds by using a dialkyl tin compound, and as performing urethane derivatization of these diol groups.

### Disclosure of the Invention

The present invention provides a novel production method for synthesizing more easily a 7-position urethane derivative having an excellent antitumor effect, by using a 12-membered ring macrolide compound represented by Formula (1) as a raw material, and provides a production intermediate thereof.

For achieving the above purpose, present inventors have worked hard to find that, by acetalizing hydroxyl groups at 6- and 7-positions of the 12-membered ring macrolide compound represented by Formula (1) with dialkyl tin (IV) oxide and, after that, reacting the product with a carbamoyl halide derivative, the 7-position urethane derivative being the target can effectively be produced, while not necessarily protecting hydroxyl groups at other positions. The present invention is based on such knowledge.

Therefore, according to the present invention, inventions [1] to [9] below are provided.
[1] A method for producing a macrolide derivative represented by Formula (5) 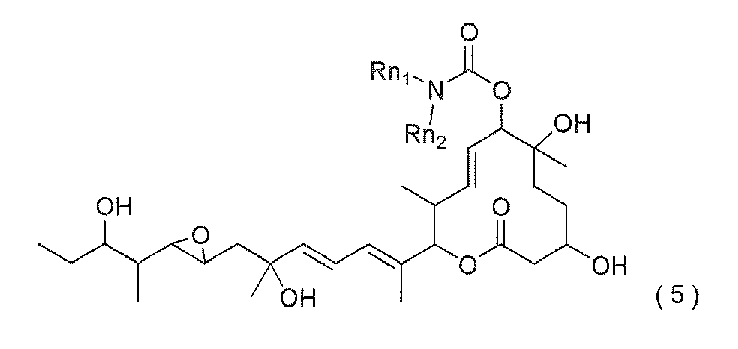 (where Rn₁ and Rn₂ may be the same or different, and represent
   a) a hydrogen atom,
   b) a C1 to C22 alkyl group that may have substituent(s),
   c) an unsaturated C2 to C22 alkyl group that may have substituent(s),
   d) a C6 to C14 aryl group that may have substituent(s),
   e) a 5- to 14-membered ring heteroaryl group that may have substituent(s),
   f) a C7 to C22 aralkyl group that may have substituent(s),
   g) a 5- to 14-membered ring heteroaralkyl group that may have substituent(s),
   h) a C3 to C14 cycloalkyl group that may have substituent(s),
   i) a 3- to 14-membered non-aromatic heterocyclic group that may have substituent(s), or
   j) a 3- to 14-membered non-aromatic heterocyclic group formed by Rn₁ and Rn₂ with a nitrogen atom that is bonded together (the 3- to 14-membered non-aromatic heterocyclic group may have substituent(s))),
   comprising a step of reacting a tin (IV)-containing macrolide derivative represented by Formula (3) 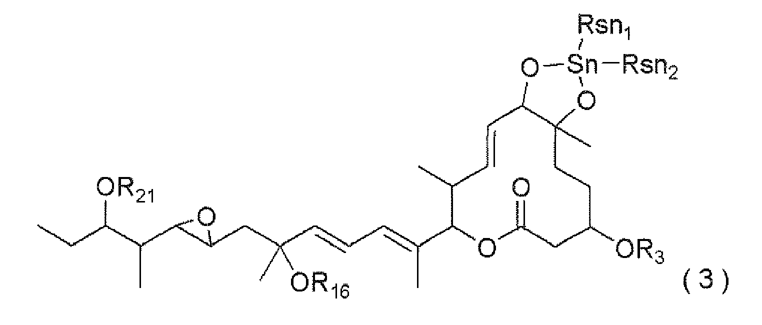 (where each of R₃, R₁₆ and R₂₁ independently represents a hydrogen atom or a protective group of hydroxyl group, and each of Rsn₁ and Rsn₂ independently represents a C1 to C30 alkyl group) in a solvent with a carbamoyl halide derivative represented by Formula (4) 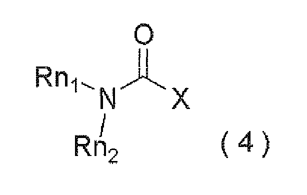 (where Rn₁ and Rn₂ are defined in the same way as above, and X represents a halogen atom) and, subsequently where necessary, subjecting the product to an elimination reaction of a protective group of hydroxyl group.
[2] The production method according to [1] further comprising a step of reacting a macrolide derivative represented by Formula (1) 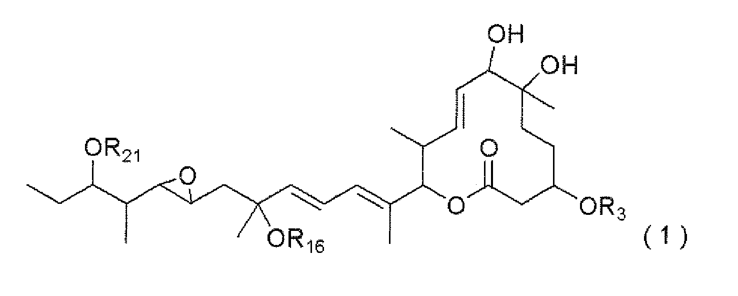 (where each of R₃, R₁₆ and R₂₁ independently represents a hydrogen atom or a protective group of hydroxyl group) in a solvent with a dialkyl tin (IV) oxide represented by Formula (2) 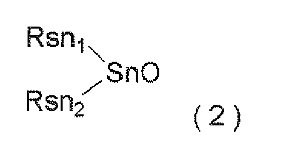 (where each of Rsn₁ and Rsn₂ independently represents a C1 to C30 alkyl group), before reacting the tin (IV)-containing macrolide derivative represented by Formula (3) with the carbamoyl halide derivative represented by Formula (4).
[3] The production method according to [1] or [2], wherein the carbamoyl halide derivative represented by Formula (4) is a derivative represented by Formula (4-1) 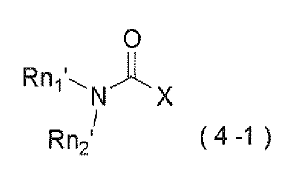 (where Rn₁' and Rn₂' form a 3- to 14-membered non-aromatic heterocyclic group with a nitrogen atom bonded together (the 3- to 14-membered non-aromatic heterocyclic group may have substituent(s)), and X represents a halogen atom).
[4] The production method according to [1] or [2], wherein the carbamoyl halide derivative represented by Formula (4) is a derivative represented by Formula (4-2). 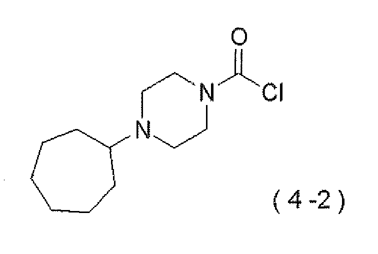
[5] A method for producing a tin (IV)-containing macrolide derivative represented by Formula (3) 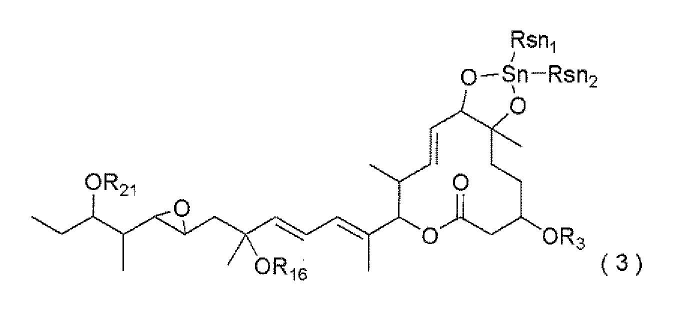 (where each of R₃, R₁₆ and R₂₁ independently represents a hydrogen atom or a protective group of hydroxyl group, and each of Rsn₁ and Rsn₂ independently represents a C1 to C30 alkyl group), comprising a step of reacting a macrolide derivative represented by Formula (1) 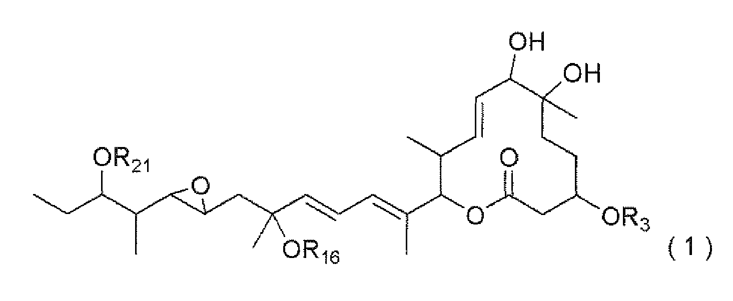 (where each of R₃, R₁₆ and R₂₁ independently represents a hydrogen atom or a protective group of hydroxyl group) in a solvent with a dialkyl tin (IV) oxide represented by Formula (2) 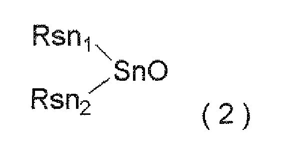 (where each of Rsn₁ and Rsn₂ independently represents a C1 to C30 alkyl group).
[6] A tin (IV)-containing macrolide derivative represented by Formula (3) 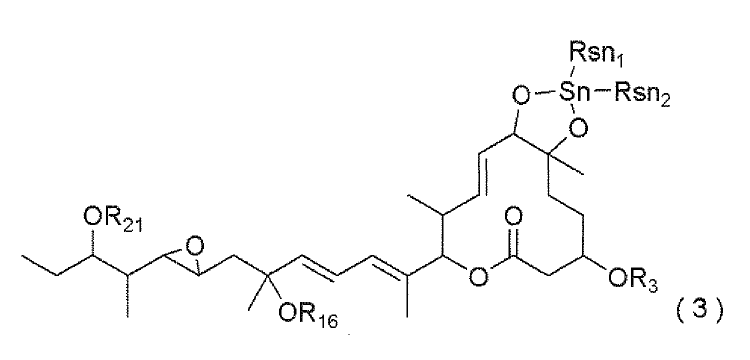 (where each of R₃, R₁₆ and R₂₁ independently represents a hydrogen atom or a protective group of hydroxyl group, and each of Rsn₁ and Rsn₂ independently represents a C1 to C30 alkyl group).
[7] The tin (IV)-containing macrolide derivative according to [6], wherein R₃, R₁₆ and R₂₁ are hydrogen atoms.
[8] The tin (IV)-containing macrolide derivative according to [6], wherein each of Rsn₁ and Rsn₂ independently represents a C1 to C10 alkyl group.
[9] The carbamoyl halide derivative represented by Formula (4-2).

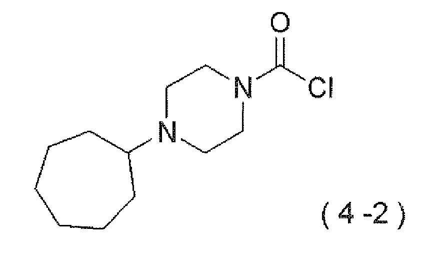

In the present invention, the tin (IV)-containing macrolide derivative represented by Formula (3) may be one that is produced by the method described in [5].

Further, the present invention provides the macrolide derivative represented by Formula (3) obtained by the production method of [5].

### Detailed Description of the Invention

Hereinafter, the meaning of terms, symbols and the like described herein will be explained, and the present invention will be explained in detail.

In the present Description, a structural formula of a compound occasionally represents certain isomers, as a matter of convenience. All the isomers such as geometric isomers, optical isomers based on an asymmetric carbon, rotational isomers, stereoisomers, and tautomers which are generated on the basis of the structure of the compound, and isomer mixtures are included in the present invention, which are not limited to expediential description of a formula and may be either one of isomers or a mixture thereof. Accordingly, the compound of the present invention has multiple asymmetric carbon atoms in the molecule to thereby be able to provide various diastereomers, and is not limited to any of them.

On "the protective group of a hydroxyl group" used herein, no limitation is imposed as long as the hydroxyl group can be protected. Examples thereof include a 1-ethoxyethyl group, a tetrahydropyranyl group, a 1-methoxycyclohexyl group, a 4-methoxytetrahydropyranyl group, a 4-methoxytetrahydrothiopyranyl group, a 4-methoxytetrahydrothiopyranyl-S,S-dioxide group, a tert-butyldimethylsilyl group, a triethylsilyl group, a diethylisopropylsilyl group, a trimethylsilyl group, a triisopropylsilyl group, a di-tert-butylmethylsilyl group, a diphenylmethylsilyl group, and the like.

"The C1 to C10 alkyl group" used herein represents a linear or branched alkyl group having 1 to 10 carbon atoms. Examples thereof include a methyl group, an ethyl group, a n-propyl group, an iso-propyl group, a n-butyl group, an iso-butyl group, a sec-butyl group, a tert-butyl group, a n-pentyl group, a 1,1-dimethylpropyl group, a 1,2-dimethylpropyl group, a 2,2-dimethylpropyl group, a 1-ethylpropyl group, a 1-methylbutyl group, a 2-methylbutyl group, a n-hexyl group, a 1-ethyl-2--methylpropyl group, a 1,1,2-trimethylpropyl group, a 1,1-dimethylbutyl group, a 1,2-dimethylbutyl group, a 2,2-dimethylbutyl group, a 1,3-dimethylbutyl group, a 2, 3-dimethylbutyl group, a 1-ethylbutyl group, a 2-ethylbutyl group, a 2-methylpentyl group, a 3-methylpentyl group, a n-heptyl group, a n-octyl group, a n-nonyl group, a n-decyl group, and the like.

The "C1 to C22 alkyl group" used herein represents a linear or branched alkyl group having 1 to 22 carbon atoms. Examples thereof include, in addition to examples of the C1 to C10 alkyl group, an undecanyl group, a dodecanyl group, a tridecanyl group, a tetradecanyl group, a pentadecanyl group, a hexadecanyl group, a heptadecanyl group, an octadecanyl group, a nonadecanyl group, an icosanyl group, a henicosanyl group, a docosanyl group, and the like.

"The C1 to C30 alkyl group" used herein represents a linear or branched alkyl group having 1 to 30 carbon atoms, including, in addition to examples of the C1 to C22 alkyl group, a tricosanyl group, a pentacosanyl group, a hexacosanyl group, a nonacosanyl group, a triacontanyl group, and the like.

"The unsaturated C2 to C22 alkyl group" used herein represents a linear or branched alkenyl group having 2 to 22 carbon atoms, or a linear or branched alkynyl group having 2 to 22 carbon atoms. Examples thereof may include a vinyl group, an allyl group, a 1-propenyl group, an isopropenyl group, a 2-methyl-1-propenyl group, a 2-methyl-2-propenyl group, a 1-butenyl group, a 2-butenyl group, a 3-butenyl group, a 1-pentenyl group, a 1-hexenyl group, a 1,3-hexadienyl group, a 1,5-hexadienyl group, an ethynyl group, a 1-propynyl group, a 2-propynyl group, a 1-butynyl group, a 2-butynyl group, a 3-butynyl group, a 1-ethynyl-2-propynyl group, a 2-methyl-3-butynyl group, a 1-pentynyl group, a 1-hexynyl group, a 1,3-hexadiynyl group, a 1,5-hexadiynyl group, and the like.

"The C6 to C14 aryl group" used herein means an aromatic hydrocarbon cyclic group constituted of 6 to 14 carbon atoms, including a monocyclic group, and condensed rings such as a bicyclic group and a tricyclic group. Examples thereof may include a phenyl group, an indenyl group, a 1-naphthyl group, a 2-naphthyl group, an azulenyl group, a heptalenyl group, an indacenyl group, an acenaphthylenyl group, a fluorenyl group, a phenalenyl group, a phenanthryl group, an anthryl group, and the like.

"The 5- to 14-membered ring heteroaryl group" herein means a monocyclic, bicyclic or tricyclic 5- to 14-membered aromatic heterocyclic group constituted by containing one or more hetero-atoms selected from the group consisting of a nitrogen atom, a sulfur atom and an oxygen atom. Examples of nitrogen-containing aromatic heterocyclic groups may include a pyrrolyl group, a pyridyl group, a pyridazinyl group, a pyrimidinyl group, a pyrazinyl group, a triazolyl group, a tetrazolyl group, a benzotriazolyl group, a pyrazolyl group, an imidazolyl group, a benzimidazolyl group, an indolyl group, an isoindolyl group, an indolizinyl group, a purinyl group, an indazolyl group, a quinolyl group, an isoquinolyl group, a quinolizinyl group, a phthalazinyl group, a naphthyridinyl group, a quinoxalyl group, a quinazolinyl group, a cinnolinyl group, a pteridinyl group, an imidazotriazinyl group, a pyrazinopyridazinyl group, an acridinyl group, a phenanthridinyl group, a carbazolyl group, a carbazolinyl group, a perimidinyl group, a phenanthrolinyl group, a phenazinyl group, an imidazopyridyl group, an imidazopyrimidinyl group, a pyrazolopyridyl group, a pyrazolopyrimidinyl group, and the like. Examples of sulfur-containing aromatic heterocyclic groups may include a thienyl group, a benzothienyl group and the like, and examples of oxygen-containing aromatic heterocyclic groups may include a furyl group, a pyranyl group, a cyclopentapyranyl group, a benzofuranyl group, an isobenzofuranyl group and the like, and examples of aromatic heterocyclic groups containing two or more different kinds of hetero atoms may include a thiazolyl group, an isothiazolyl group, a benzothiazolyl group, a benzthiadiazolyl group, a phenothiazinyl group, an isoxazolyl group, a furazanyl group, a phenoxazinyl group, an oxazolyl group, an isoxazoyl group, a benzoxazolyl group, an oxadiazolyl group, a pyrazoloxazolyl group, an imidazothiazolyl group, a thienofuranyl group, a furopyrrolyl group, a pyridoxazinyl group and the like.

"The C7 to C22 aralkyl group" used herein means a group in which a substitutable part in "the C1 to C22 alkyl group" as defined above is substituted by "the C6 to C14 aryl group" as defined above. Specific examples thereof may include a benzyl group, a phenethyl group, a 3-phenylpropyl group, a 4-phenylbutyl group, a 1-naphthylmethyl group, a 2-naphthylmethyl group, and the like.

"The 5- to 14-membered ring heteroaralkyl group" used herein means a group in which a substitutable part in "the C1 to C22 alkyl group" as defined above is substituted by "the 5-to 14-membered ring heteroaryl group" as defined above. Specific examples thereof may include a thienylmethyl group, a furylmethyl group, a pyridylmethyl group, a pyridazylmethyl group, a pyrimidylmethyl group, a pyrazylmethyl group, and the like.

"The C3 to C14 cycloalkyl group" used herein means a cycloalkyl group constituted of 3 to 14 carbon atoms. Specific examples thereof may include a cyclopropyl group, a cyclobutyl group, a cyclopentyl group, a cyclohexyl group, a cycloheptyl group, a cyclooctyl group, and the like.

"The 3- to 14-membered non-aromatic heterocyclic group" used herein means a monocyclic, bicyclic or tricyclic 3- to 14-membered non-aromatic heterocyclic group that may include one or more hetero atoms selected from the group consisting of a nitrogen atom, a sulfur atom and an oxygen atom. Examples thereof may include an aziridinyl group, an azetidyl group, a pyrrolidinyl group, a pyrrolyl group, a piperidyl group, a piperazinyl group, a homopiperidinyl group, a homopiperazinyl group, an imidazolyl group, a pyrazolidyl group, an imidazolidinyl group, a morpholinyl group, a thiomorpholinyl group, an imidazolinyl group, an oxazolinyl group, a quinuclidinyl group, a tetrahydrofuranyl group, a tetrahydrothienyl group, and the like. The non-aromatic heterocyclic group also includes groups that are induced from a pyridone ring, and non-aromatic condensed rings (for example, groups induced from a phthalimide ring, a succinimide ring or the like).

The substituent of "that may have substituent(s)" used herein includes one or more groups selected from:
(1) a halogen atom,
(2) a hydroxyl group,
(3) a thiol group,
(4) a nitro group,
(5) a nitroso group,
(6) a cyano group,
(7) a carboxyl group,
(8) a hydroxysulfonyl group,
(9) an amino group,
(10) a C1 to C22 alkyl group (such as a methyl group, an ethyl group, a n-propyl group, an iso-propyl group, a n-butyl group, an iso-butyl group, a sec-butyl group or a tert-butyl group),
(11) an unsaturated C2 to C22 alkyl group (such as a vinyl group, an allyl group, a 1-propenyl group, an isopropenyl group, an ethynyl group, a 1-propynyl group, a 2-propynyl group, a 1-butynyl group, a 2-butynyl group or a 3-butynyl group),
(12) a C6 to C14 aryl group (such as a phenyl group, a 1-naphthyl group or a 2-naphthyl group),
(13) a 5- to 14-membered ring heteroaryl group (such as a thienyl group, a furyl group, a pyridyl group, a pyridadinyl group, a pyrimidinyl group or a pyrazinyl group),
(14) a 3- to 14-membered non-aromatic heterocyclic group (such as an aziridinyl group, an acetidyl group, a pyrrolidinyl group, a pyrrolyl group, a piperidyl group, a piperazinyl group, an imidazolyl group, a pyrazolidyl group, an imidazolidinyl group, a morpholinyl group, a thiomorpholinyl group, an imidazolinyl group, an oxazolinyl group or a quinuclidinyl group),
(15) a C3 to C8 cycloalkyl group (such as a cyclopropyl group, a cyclobutyl group, a cyclopentyl group, a cyclohexyl group, a cycloheptyl group or a cyclooctyl group),
(16) a C1 to C22 alkoxy group (such as a methoxy group, an ethoxy group, a n-propoxy group, an iso-propoxy group, a n-butoxy group, an iso-butoxy group, a sec-butoxy group or a tert-butoxy group),
(17) an unsaturated C2 to C22 alkoxy group (such as a vinyloxy group, an allyloxy group, a 1-propenyloxy group, an isopropenyloxy group, an ethynyloxy group, a 1-propynyloxy group, a 2-propynyloxy group, a 1-butynyloxy group or a 2-butynyloxy group),
(18) a C6 to C14 aryloxy group (such as a phenoxy group, a 1-naphthyloxy group or a 2-naphthyloxy group),
(19) a C7 to C22 aralkyloxy group (such as a benzyloxy group, a phenethyloxy group, a 3-phenylpropyloxy group, a 4-phenylbutyloxy group, a 1-naphthylmethyloxy group or a 2-naphthylmethyloxy group)
(20) a 5- to 14-membered ring heteroaralkyloxy group (such as a thienylmethyloxy group, a furylmethyloxy group, a pyridylmethyloxy group, a pyridazinylmethyloxy group, a pyrimidinylmethyloxy group or a pyrazinylmethyloxy group)
(21) a 5- to 14-membered ring heteroaryloxy group (such as a thienyloxy group, a furyloxy group, a pyridyloxy group, a pyridazyloxy group, a pyrimidyloxy group or a pyrazyloxy group),
(22) an aliphatic C1 to C22 acyl group (such as an acetyl group, a propionyl group, a butyryl group, an iso-butyryl group, a valeryl group, an iso-valeryl group, a pivaloyl group, a caproyl group, a decanoyl group, a lauroyl group, a myristoyl group, a palmitoyl group, a stearoyl group, an arachidoyl group, an acrylic group, a propioloyl group, a crotonoyl group, an iso-crotonoyl group, an oleoyl group or linolenoyl group),
(23) an aromatic C7 to C22 acyl group (such as a benzoyl group, a 1-naphthoyl group or a 2-naphthoyl group),
(24) an aliphatic C2 to C22 acyloxy group (such as an acetoxy group, a propionyloxy group or an acryloxy group),
(25) a C2 to C22 alkoxycarbonyl group (such as a methoxycarbonyl group, an ethoxycarbonyl group, a n-propoxycarbonyl group, an iso-propoxycarbonyl group, a n-butoxycarbonyl group, an iso-butoxycarbonyl group, a sec-butoxycarbonyl group or a tert-butoxycarbonyl group),
(26) an unsaturated C3 to C22 alkoxycarbonyl group (such as a vinyloxycarbonyl group, an allyloxycarbonyl group, a 1-propenyloxycarbonyl group, an isopropenyloxycarbonyl group, a propargyloxycarbonyl group or a 2-butynyloxycarbonyl group),
(27) a C1 to C22 alkylthio group (such as a methylthio group, an ethylthio group, a n-propylthio group or an iso-propylthio group),
(28) a C1 to C22 alkylsulfinyl group (such as a methylsulfinyl group, an ethylsulfinyl group, a n-propylsulfinyl group or an iso-propylsulfinyl group),
(29) a C1 to C22 alkylsulfonyl group (such as a methylsulfonyl group, an ethylsulfonyl group, a n-propylsulfonyl group or an iso-propylsulfonyl group),
(30) a C6 to C14 arylsulfonyl group (such as a benzenesulfonyl group, a 1-naphthalenesulfonyl group or a 2-naphthalenesulfonyl group),
(31) a C1 to C22 alkylsulfonyloxy group (such as a methylsulfonyloxy group, an ethylsulfonyloxy group, a n-propylsulfonyloxy group or an iso-propylsulfonyloxy group), and
(32) a carbamoyl group.

In addition, the (9) amino group and (32) carbamoyl group mentioned as the substituent in "that may have substituent (s) " may further be substituted by one or two C1 to C22 alkyl groups, unsaturated C2 to C22 allyl groups or C6 to C14 aryl groups.

"The halogen atom" used herein means a fluorine atom, a chlorine atom, a bromine atom or an iodine atom.

According to the production method of the present invention that goes through the tin (IV)-containing macrolide derivative represented by the above-mentioned Formula (3) as the production intermediate, from the 12-membered ring macrolide compound being a raw material represented by Formula (1), the 7-position urethane derivative that is represented by Formula (5) and has an excellent antitumor effect can be produced in a short production process with a good efficiency.

### Reaction process 1

Process of producing the tin (IV)-containing macrolide derivative of Formula (3) by reacting (diols at 6- and 7-positions of) the macrolide derivative of Formula (1) with the dialkyl tin (IV) oxide of Formula (2) (Formula (1) + Formula (2) → Formula (3))

R₃, R₁₆ and R₂₁ in the macrolide derivative of Formula (1) being a raw material, independently of one another, are preferably a hydrogen atom, a 1-ethoxyethyl group, a tert-butyldimethylsilyl group or a triethylsilyl group, more preferably a hydrogen atom or a triethylsilyl group.

As an alkyl group represented by Rsn₁ or Rsn₂ in the dialkyl tin (IV) oxide of Formula (2), each independently is preferably an alkyl group having 1 to 10 carbon atoms such as a methyl group, an ethyl group, a n-butyl group, a n-octyl group or a n-decyl group, more preferably a n-butyl group or a n-octyl group.

On the solvent used in the reaction process 1, no limitation is imposed, but inert solvents that do not easily react with the raw material are desirable. Examples thereof may include alcohols such as methanol or ethanol, ethers such as tetrahydrofuran, diethyl ether, diisopropyl ether, methyl tert-butyl ether, cyclopentyl methyl ether, dioxane or dimethoxyethane, halogenated hydrocarbons such as dichloromethane, chloroform, carbon tetrachloride or 1,2-dichloroethane, hydrocarbons such as hexane, benzene or toluene, ketones such as acetone or methyl ethyl ketone, nitriles such as acetonitrile, amides such as N,N-dimethylformamide, N,N-dimethylacetamide, N-methyl-2-pyridone or hexamethylphosphorylamide, and sulfoxides such as dimethyl sulfoxide, wherein acetonitrile, tetrahydrofuran, dioxane, toluene and the like are preferable.

The reaction temperature of the reaction process 1 is from room temperature to refluxing temperature under heating of the solvent to be used, and is preferably 40 to 120°C. The reaction time is preferably 10 minutes to one day, more preferably 30 minutes to 5 hours.

In the reaction process 1, dialkyl tin (IV) oxide is used preferably at 0.3 to 5 equivalents relative to the macrolide derivative of Formula (1), more preferably at 0.5 to 1.5 equivalents. Upon the reaction, the reaction is preferably performed at the refluxing temperature of the solvent to be used, and, by removing the solvent from the reaction system under reduced pressure, the reaction progresses more effectively.

After the completion of the reaction, the solvent is distilled off under reduced pressure, which may be further purified, if necessary, by an ordinary method such as column chromatography, thin-layer chromatography, crystallization or the like. However, usually, after the completion of the reaction, a carbamoylating reaction below is continuously performed in the same reaction chamber.

Meanwhile, among macrolide derivatives of Formula (1), the derivative in which R₃, R₁₆ and R₂₁ are hydrogen atoms may be produced by the isolation and collection from a culture solution of Streptomyces sp, Mer-11107 (FERM BP-7812) (see WO-A 2002/060890), or it may also be produced by hydrolyzing the 7-position acetyl group of the macrolide derivative represented by Formula (A) that can be collected in a larger amount (see WO-A 2003/099813 and WO-A 2006/126723). It is also possible to perform a publicly known reaction for introducing a protective group of hydroxyl group for the macrolide derivative represented by Formula (A), and further to hydrolyze a 7-pasition acetyl group to produce the macrolide derivative of Formula (1) in which the protective group has been introduced.

The hydrolysis of the 7-position acetyl group can be performed according to the method described in a document (see T. W. Greene, Protective Groups in Organic Synthesis, John Wiley & Sons, (1981)) or methods pursuant to it, for example, by hydrolysis using acid or base, or chemical reduction using a hydrogenated metal complex or the like. As the base, for example, alkali metal hydroxides such as sodium hydroxide, potassium hydroxide or lithium hydroxide, alkali metal carbonates such as potassium carbonate, sodium carbonate, sodium hydrogencarbonate or cesium carbonate, guanidine, and guanidine nitrate are preferable, and a mixed salt of guanidine and guanidine nitrate is particularly preferable. The mixed salt of guanidine and guanidine nitrate may be used at 1.0 to 2.0 equivalents relative to a protective compound of the hydroxyl group of Formula (A), but, preferably, 1.0 to 1.2 equivalents are favorable. On the solvent used in the process, no particular limitation is imposed, but an inert solvent that dissolves the raw material to some extent and that does not easily react with the raw material is desirable. Examples thereof include water, alcohol-based solvents such as methanol, ethanol, isopropanol or tert-butanol, ether-based solvents such as tetrahydrofuran, diethyl ether, diisopropyl ether, dioxane, or 1,2-dimethoxyethane, nitrile-based solvents such as acetonitrile, amide-based solvents such as N,N-dimethylformamide, N,N-dimethylacetamide, N-methyl-2-pyridone or hexamethylphosphorylamide, sulfoxide-based solvents such as dimethyl sulfoxide, or mixed solvents thereof. Among these, alcohol-based solvents such as methanol or ethanol are preferable. On the reaction temperature, no particular limitation is imposed, but a temperature of 0 to 60°C is preferable, 10 to 30°C is more preferable, and 24.0 to 25.0°C is furthermore preferable. The reaction time may be 1 to 24 hours, but 3 to 10 hours is preferable.

### Reaction process 2

Process of reacting the tin (IV)-containing macrolide derivative of Formula (3) with a carbamoyl halide derivative of Formula (4) to produce the macrolide derivative of Formula (5) (Formula (3) + Formula (4) → Formula (5))

In the carbamoyl halide derivative of Formula (4), as X, above-described halogen atoms are included, and a chlorine atom or a bromine atom is preferable. As Rn₁ and Rn₂, when exemplifying them in the amine form in which Rn₁ or Rn₂ is bonded to a nitrogen atom, ones described below can be included.

Examples thereof include methylamine, ethylamine, propylamine, butylamine, octylamine, decylamine, cyclopropylamine, cyclopentylamine, cyclohexylamine, dimethylamine, diethylamine, ethylmethylamine, ethylenediamine, 1,3-propanediamine, 1,4-butanediamine, N,N-dimethylethylenediamine, N,N-dimethyl-1,3-propanediamine, N,N-dimethyl-1,4-butanediamine, N,N-diethylethylenediamine, N,N-diethyl-1,3-propanediamine, N,N-diethyl-1,4-butanediamine, N,N,N'-trimethylethylenediamine, N,N,N'-trimethyl-1,3-propanediamine, N,N,N'-trimethyl-1,4-butanediamine, N-ethyl-N',N'-dimethylethylenediamine, N-ethyl-N',N'-dimethyl-1,3-propanediamine, N-ethyl-N',N'-dimethyl-1,4-butanediamine, N,N,N'-triethylethylenediamine, N,N,N'-triethyl-1,3-propanediamine, N,N,N'-triethyl-1,4-butanediamine, N,N-diethyl-N'-methylethylenediamine, N,N-diethyl-N'-methyl-1,3-propanediamine, N,N-diethyl-N'-methyl-1,4-butanediamine, N,N'-dimethyl-N-phenylethylenediamine, N,N'-dimethyl-N-phenyl-1,3-propanediamine, N-benzyl-N,N'-dimethylethylenediamine, N-benzyl-N,N'-dimethyl-1,3-propanediamine, morpholine, thiomorpholine, thiomorpholine-S-oxide, thiomorpholine-S,S-dioxide, pyrrolidone, piperidine, piperazine, homopiperazine, 4-hydroxypiperidine, 4-methoxypiperidine, 1-methylpiperazine, 1-ethylpiperazine, 1-propylpiperazine, 1-butylpiperazine, 1-isopropylpiperazine, 1-cyclobutylpiperazine, 1-cyclopentylpiperazine, 1-cyclohexylpiperazine, 1-cycloheptylpiperazine, 1-cyclooctylpiperazine, 1-(cyclopropylmethyl)piperazine, 1-benzylpiperazine, 1-methylhomopiperazine, 1-ethylhomopiperazine, 1-(2-aminoethyl)pyrrolidine, 1-(2-(N-methylaminc)ethyl)pyrrolidine, 1-(3-aminopropyl)pyrrolidine, 1-(3-(N-methylamino)propyl)pyrrolidine, 1-(2-aminoethyl)piperidine, 1-(2-(N-methylamino)ethyl)piperidine, 1-(3-aminopropyl)piperidine, 1-(3-(N-methylamino)propyl)piperidine, 4-(2-aminoethyl)morpholine, 4-(2-(methylamino)ethyl)morpholine, 4-(3-aminopropyl)morpholine, 4-(3-(N-methylamino)propyl)morpholine, 1-(2-aminoethyl)-4-methylpiperazine, 1-(3-aminopropyl)-4-methylpiperazine, 1-(3-(N-methylamino)propyl)-4-methylpiperazine, 1-amino-4-methylpiperidine, 1-methylamino-4-methylpiperidine, 1-ethyl-4-(N-methylamino)piperidine, 1-methylamino-4-propylpiperidine, 1-butyl-4-(N-methylamino)piperidine, 1-(N,N-dimethylamino)piperidine, 1-(N,N-diethylamino)piperidine, 4-(pyrrolidin-1-yl)piperidine, 4-(piperidin-1-yl)piperidine, 3-aminoquinuclidine, 3-(N-methylamino)quinuclidine, aniline, N-methylaniline, N,N-dimethyl-p-phenylenediamine, N,N,-dimethyl-m-phenylenediamine, N,N,N'-trimethyl-p-phenylenediamine, N,N,N'-trimethyl-m-phenylenediamine, 1-naphthylamine, 2-naphthylamine, benzylamine, N-methylbenzylamine, phenethylamine, N-methylphenethylamine, 2-picolylamine, 3-picolylamine, 4-picolylamine, N-methyl-2-picolylamine, N-methyl-3-picolylamine, N-methyl-4-picolylamine, 2,5-diazabicyclo[2.2.1]heptane, 2-methyl-2,5-diazabicyclo[2.2.1]heptane, 3,8-diazabicyclo[3.2.1]octane, 1,4-diazabicyclo[4.3.0]nonane, and the like. Among these, 1-methylpiperazine, 1-isopropylpiperazine, 1-cyclohexylpiperazine, 1-cycloheptylplperazine and N,N-diethyl-N'-methylethylenediamine are preferable.

The carbamoyl halide derivative of Formula (4) can be obtained by reacting the amine exemplified as an amine formed by bonding Rn₁ and Rn₂ to a nitrogen atom, with a chlorocarbonylating agent such as triphosgene or phosgene in an inert solvent. On a solvent to be used for the reaction, no particular limitation is imposed, but an inert solvent that does not easily react with the raw material is desirable. Examples thereof may include ethers such as tetrahydrofuran, diethyl ether, diisopropyl ether, methyl tert-butyl ether, cyclopentyl methyl ether, dioxane or dimethoxyethane, halogenated hydrocarbons such as dichloromethane, chloroform, carbon tetrachloride or 1,2-dichloroethane, hydrocarbons such as hexane, benzene or toluene, ketones such as acetone or methyl ethyl ketone, and nitriles such as acetonitrile, and methyl tert-butyl ether, acetonitrile, tetrahydrofuran, dioxane, toluene, chloroform and the like are preferable.

The reaction time of the amine and chlorocarbonylating agent is preferably 10 minutes to 5 days, more preferably 12 to 36 hours. The reaction temperature is -20°C to the boiling point of a solvent to be used, preferably -5 to 40°C.

As the reaction ratio of the amine and chlorocarbonylating agent, the use of 0.8 to 10 equivalents of the chlorocarbonylating agent relative to one equivalent of the amine is preferable, and the use of 0.9 to 2 equivalents is more preferable. After the completion of the reaction, the intended carbamoyl halide derivative is collected from the reaction mixture according to an ordinary method. For example, when an insoluble matter exists, the derivative can be obtained by appropriately performing filtration and distilling off the solvent under reduced pressure, or by diluting off the reaction mixture with an organic solvent such as methyl tert-butyl ether, toluene or ethyl acetate and washing the same, drying the organic layer over anhydrous sodium sulfate or the like, and, after that, distilling off the solvent, which may further be purified, if necessary, by an ordinary method such as column chromatography, thin layer chromatography, high-performance liquid chromatography or crystallization.

As the carbamoyl halide derivative of Formula (4), the derivative represented by Formula (4-1) 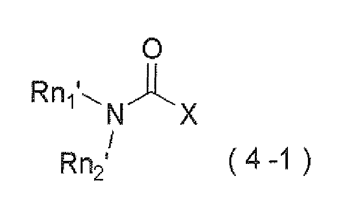 (where, Rn₁' and Rn₂' represent a 3- to 14-membered neon-aromatic heterocyclic group formed with a nitrogen atom that is bonded together (the 3- to 14-membered non-aromatic heterocyclic group may have substituent(s)), and X represents a halogen atom) is preferable, and, in particular, the carbamoyl halide derivative represented by Formula (4-2) below is an extremely useful intermediate for effectively producing an active macrolide derivative of Formula (5).

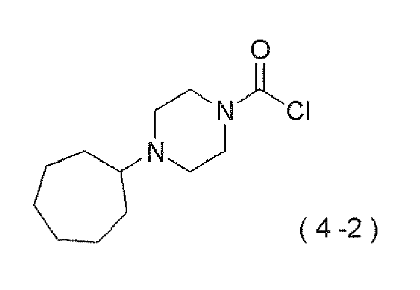

On the solvent used in the reaction process 2, no particular limitation is imposed, but an inert solvent that does not easily react with the tin (IV)-containing macrolide derivative of Formula (3) and the carbamoyl halide derivative of Formula (4) is desirable. Examples thereof include ethers such as tetrahydrofuran, diethyl ether, diisopropyl ether, methyl tert-butyl ether, cyclopentyl methyl ether, dioxane or dimethoxyethane, halogenated hydrocarbons such as dichloromethane, chloroform, carbon tetrachloride or 1,2-dichloroethane, hydrocarbons such as hexane, benzene or toluene, ketones such as acetone or methyl ethyl ketone, nitriles such as acetonitrile, amides such as N,N-dimethylformamide, N,N-dimethylacetamide, N-methyl-2-pyridone or hexamethylphosphorylamide, and sulfoxides such as dimethylsulfoxide. Acetonitrile, tetrahydrofuran, dioxane, toluene and the like are preferable.

The reaction temperature of the reaction process 2 is 0°C to the refluxing temperature under heating of the solvent to be used, and is preferably 5 to 90°. The reaction time is preferably 10 minutes to 5 days, more preferably 30 minutes to 48 hours.

In the reaction process 2, the carbamoyl halide derivative of Formula (4) is used preferably at 1 to 10 equivalents relative to the tin (IV)-containing macrolide derivative of Formula (3), more preferably 1 to 4 equivalents.

In the reaction process 2, when the reaction rate is low only by the addition of the carbamoyl halide derivative of Formula (4), it is possible to heighten the reaction rate by heating or adding a base, or by both operations. As the base, organic bases or the like are included, and, for example, diisopropylethylamine, 4-dimethylaminopyridine, triethylamine, pyridine, 2, 6-lutidine or the like is preferably used.

In order to check the completion of the reaction, a part of the reaction liquid is suspended in acetonitrile, which is filtrated and then subjected to HPLC analysis, and the instant, when the area dimension of the macrolide of Formula (5), or of a macrolide derivative having a protective group of hydroxyl group at 3-position, 16-position and/or 21-position becomes constant, is determined to be the completion of the reaction.

After the completion of the reaction, where necessary, that is, when R₃, R₁₆ and/or R₂₁ is a protective group, the obtained reaction product is subjected to an elimination reaction of the protective group of hydroxyl group to give the macrolide derivative of Formula (5).

The process is achieved by performing a deprotection treatment as shown below for the obtained compound in an inert solvent. The deprotection reaction of the protective group of hydroxyl group differs depending on the kind of the protective group, and can be performed by a method well known in synthetic organic chemistry.

For example, the deprotection of respective hydroxyl groups such as 1-ethoxyethyl, tetrahydropyranyl, 1-methoxycyclohexyl, 4-methoxytetrahydropyranyl, 4-methoxytetrahydrothiopyranyl and 4-methoxytetrahydrothiopyranyl-S,S-dioxide is easily performed by an acid treatment in an inert solvent. Examples of acids may include organic acids such as pyridinium p-toluene sulfonate (PPTS), p-toluene sulfonic acid, camphor sulfonic acid, acetic acid, trifluoroacetic acid or methane sulfonic acid, and inorganic acids such as hydrogen chloride, nitric acid, hydrochloric acid or sulfuric acid. Preferable are, for example, pyridinium p-toluene sulfonate, p-toluene sulfonic acid, camphor sulfonic acid and the like. On the solvent to be used for the reaction, no limitation is imposed, but one that does not easily react with the raw materials is desirable. For example, alcohol-based solvents such as methanol, ethanol, isopropanol or tert-butanol are preferable, and these and the above-described inert solvent may also be used in mixture. The amount of the acid used for the reaction is 0.5 to 5 equivalents relative to the macrolide derivative having the protective group, preferably 1 to 3 equivalents. The reaction time is 10 minutes to 10 days, preferably 1 day to 4 days. The reaction temperature is -78°C to the reflexing temperature under heating, preferably -10°C to 50°C.

When it is protected by other protective groups such as tert-butyldimethylsilyl, triethylsilyl, diethylisopropyllsilyl, trimethylsilyl, triisopropylsilyl, di-tert-butylmethylsilyl and diphenylmethylsilyl, the deprotection is possible by, for example, a treatment with a fluorine anion or acid. Examples of the fluorine anion may include tetrabutylammonium fluoride, hydrogen fluoride, potassium fluoride, pyridinium hydrogen fluoride and the like, and examples of the acid may include the above-described organic acid, inorganic acids and the like. Preferable examples include acetic acid, formic acid, trifluoroacetic acid, pyridinium p-toluene sulfonate, camphor sulfonic acid and the like. On the solvent used for the reaction, no particular limitation is imposed, but one that does not easily react with the raw material is desirable, and the above-described inert solvents are included. For example, tetrahydrofuran, diethyl ether, water or the like is preferably used. The amount of the fluorine anion and the acid used for the reaction are 1 to 5 equivalents and 0.5 to 5 equivalents, respectively, relative to the macrolide derivative having the protective group, preferably 1 to 4 equivalents and 0.5 to 3 equivalents, respectively. The reaction time is 10 minutes to 30 hours, preferably 1 to 2 hours. The reaction temperature is -78°C to the refluxing temperature under heating, preferably -10°C to 50°C.

After the completion of the reaction, the intended macrolide derivative represented by Formula (5) is collected from the reaction mixture according to an ordinary method. For example, when an insoluble matter exists, the derivative can be obtained by appropriately filtering off and distilling off the solvent under reduced pressure, or by diluting the reaction mixture with an organic solvent such as ethyl acetate and washing the same with water, drying the organic layer over anhydrous sodium sulfate or the like, and, after that, distilling off the solvent, which may further be purified, if necessary, by an ordinary method such as column chromatography, thin layer chromatography, high-performance liquid chromatography or crystallization.

### Examples

Hereinafter, the present invention will be described based on examples. But, the present invention is not limited to the embodiments below.

### Example 1

### Synthesis of (8E,12E,14E)-3,6,7,16,21-pentahydroxy-6,10,12,16,20-pentamethyl-18,19-epoxytricosa-8,12,14-trien-11-olide

### (Reference Example)

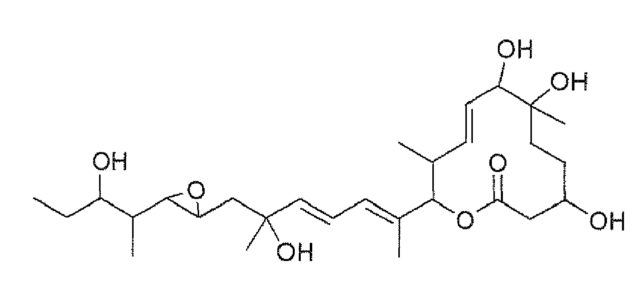

To a methanol solution (41.3%) of (8E,12E,14E)-7-acetoxy-6,10,12,16,20-pentamethyl-3,6,16,21-tetrahyciroxy-18,19-epoxytricosa-8,12,14-trien-11-olide (1.92 g, 3.47 mmol)), methanol (19 ml) was added and stirred, to which potassium carbonate (0.72 g, 5.21 mmol) was added and stirred for 1 hour under cooling with water. The reaction mixture was diluted with ethyl acetate (95 ml), and washed twice with a 25% salt solution (50 ml). The obtained organic layer was dried over sodium sulfate, filtrated and then concentrated under reduced pressure to give the crudely purified subject compound (2.29 g) as white powder.

### Example 2

### Synthesis of 4-cycloheptylpiperazine carbonyl chloride

### (Example)

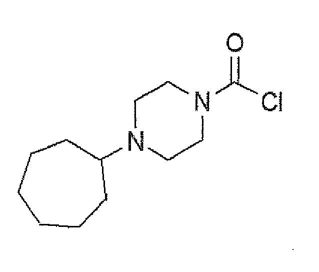

To a solution of 1-cycloheptylpiperazine 100 mg (0.549 mmol) in methyl-tert-butyl ether (MTBE) 5 ml, triphosgene 162 mg (0.549 mmol) was added and stirred over night under ice cooling. After the addition, white precipitate was generated. The reaction solution was diluted with MTBE, and the MTBE solution was washed twice with saturated sodium bicarbonate water and was then dried over anhydrous sodium sulfate. The solvent was removed to give a pale yellow solid 122.8 mg. By TLC (hexane:ethyl acetate = 2:1), slight impurities were confirmed, and, therefore, the pale yellow solid was dissolved again in MTBE, which was washed twice with saturated sodium bicarbonate water and then dried over anhydrous sodium sulfate. The solvent was removed to give a white solid of 4-cycloheptylpiperazine carbonyl chloride 100.8 mg (yield: 75.1%).

Rf value 0.57 (TLC; Merck 1.05715, developing solvent; hexane:ethyl acetate = 2:1)
¹³C-NMRSpectrum(CDCl₃,125MHz)δ(ppm):148.28,65.13,49.30,48.47, 47.86,46.90,30.01,28.03,25.54.
¹H-NmRspectrum(CDCl₃,500MHz)δ(ppm):1.35-1.60(m,8H), 1.64-1.70(m,2H),1.78-1.81(m,2H),2.56(brs,5H),3.61(brs,2H), 3.70(brs,2H)
ESI-MS m/z 245(M+H)⁺.

### Example 3

### Synthesis of (8E,12E,14E)-6,7-(dibutylstannylene)dioxy-6,10,12,16,20-pentamethyl-3,16,21-trihydroxy-18,19-epoxytricosa-8,12,14-trien-11-olide (Example)

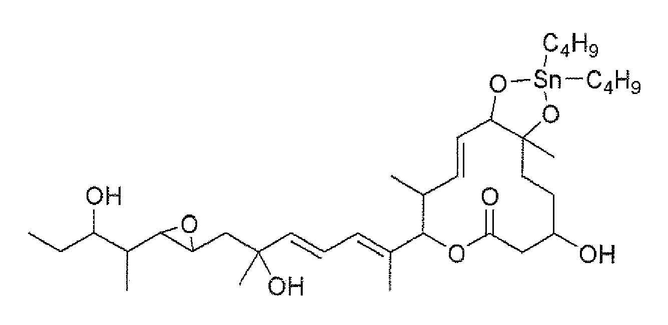

To a solution of crudely purified (8E,12E,14E)-3,6,7,16,21-pentahydroxy-6,10,12,16,20-pentame thyl-18,19-epoxytricosa-8,12,14-trien-11-olide (0.18 g, 0.35 mmol) in a mixture of tetrahydrofuran (4 ml) and toluene (20 ml), dibutyl tin (IV) oxide (97.0mg, 0.39mmol) was added. The mixture was refluxed under heating for 4 hours (at this time, the system was set so that the cooled solvent was dehydrated with calcium chloride and returned to the reaction solution). After cooling the reaction mixture to room temperature, it was concentrated under reduced pressure to give the subject compound as a pale yellow oil.
¹H-NMRSpectrum(CDCl₃,500MHz)δ(ppm):0.88-1.00(16H,m), 1.01-1.78(16H,m),1.12(3H,s),1.64(3H,s),1.77(3H,s), 2.02-2.08(1H,m),2.42-2.58(3H,m),2.74-2.78(1H,m), 2.96-3.00(1H,m),3.45(1H,d,J=10.4Hz),3.62-3.76(3H,m), 5.11(1H,d,J=10.5Hz),5.27(1H,dd,J=9.7,15.2Hz), 5.45(1H,dd,J=9.6,15.3Hz),5.85(1H,d,J=15.1Hz), 6.09-6.14(1H,m),6.53(1H,dd,J=11.2,14.9Hz).

### Example 4

### Synthesis of (8E,12E,14E)-7-((4-cycloheptylpiperazin-1-yl)carbonyl)oxy-6,10,12,16,20-pentamethyl-3,6,16,21-tetrahydroxy-18,19-epoxytricosa-8,12,14-trien-11-olide (Example)

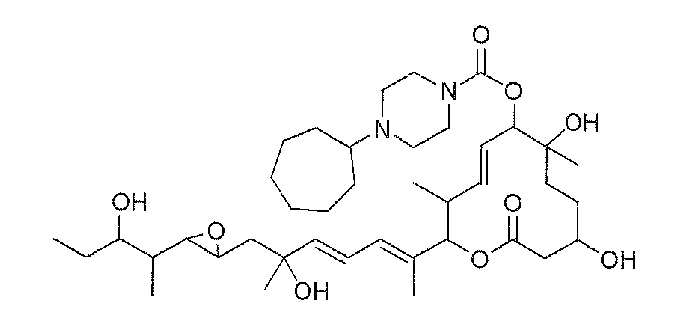

The crudely purified ((8E,12E,14E)-6,7-(dibutylstannylene)dioxy-6,10,12,16,20-pentamethyl-3,16,21-trihydroxy-18,19-epoxytricosa-8,12,14-trien-11-olide obtained in Example 3 was suspended and dissolved in toluene (9.3 ml), to which triethylamine (0.17 ml, 1.20 mmol) and 4-cycloheptylpiperazine carbonyl chloride (0.17 g, 0.69 mmol) were added. The mixture was stirred at 70 to 80°C for 40 hours. The reaction mixture was cooled to room temperature and concentrated under reduced pressure and then methanol (10 ml) was added to form a solution. The solution was measured by HPLC analysis (Chemicals Evaluation and Research Institute, L-column ODS, 4.6 × 250 mm, mobile phase; acetonitrile/(1.5 mM potassium dihydrogen phosphate + 50 mM sodium perchlorate) = 35/65, flow rate; 1 ml/min, detection wavelength; 241 nm, column temperature; a constant temperature near 23°C) to confirm the subject compound (retention time; 14.6 min, relative purity;
75.9%).

### Example 5

### Synthesis of (8E,12E,14E)-7-((4-cycloheptylpiperazin-l-yl)carbonyl)oxy-6,10,12,16,20-pentamethyl-3,6,16,21-tetrahydroxy-18,19-epoxytricosa-8,12,14-trien-11-olide (Example)

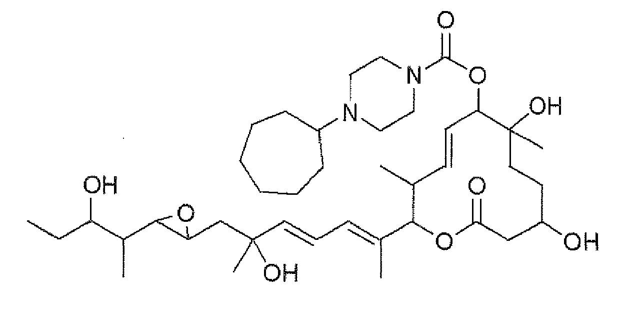

To crudely purified (8E,12E,14E)-3,6,7,16,21-pentahyciroxy-6,10,12,16,20-pentamethyl-18,19-epoxytricosa-8,12,14-trien-11-olide (0.74 g, 1.4 mmol) in a mixed liquid of tetrahydrofuran (4 ml) and toluene (15 ml), dioctyl tin (IV) oxide (0.54 g, 1.5 mmol) was added. The mixture was stirred at 90°C for 30 minutes. The solution was concentrated (exterior temperature 90°C) to about 5 ml under reduced pressure, to which toluene (15 ml) was added and the solution further concentrated to about 5 ml under reduced pressure (exterior temperature 90°C). After cooling the solution to room temperature, tetrahydrofuran (11 ml), 4-dimethylaminopyridine (0.18 g, 1.5 mmol) and 4-cycloheptylpiperazine carbonyl chloride (0.73 g, 3.0 mmol) were added. The mixture was stirred at room temperature for 25 hours. To the reaction mixture, toluene (12 ml) and tetrahydrofuran (4 ml) were added. The mixture was washed with a 5% ammonium chloride aqueous solution (15 ml) twice, and with 25% salt water (15 ml). The obtained organic layer was dried over sodium sulfate, filtrated and then concentrated under reduced pressure to give a crudely purified subject compound (0.87 g, 1.2 mmol) as a yellow product (5.06 g).

### Example 6

### Synthesis of (8E,12E,14E)-7-((4-methylpiperazin-1-yl)-carbonyl)oxy-6,10,12,16,20-pentamethyl-3,6,16,21-tetrahydroxy-18,19-epoxytricosa-8,12,14-trien-11-olide

### (Example)

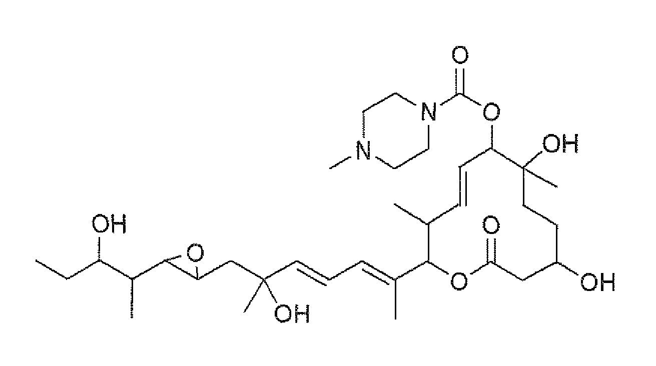

The subject compound (colorless oil) was synthesized in the same manner as Example 4, except for using 4-methylpiperazine carbonyl chloride hydrochloride in place of 4-cycloheptylpiperazine carbonyl chloride.
¹H-NMRSpectrum(CD₃OD,400MHz)δ(ppm):0.89(3H,d,J=6.8Hz),0.90 (3H,d,J=6.8Hz),0.94(3H,t,J=7.6Hz),1.19-1.28(4H,m),1.32-1.68 (10H,m),1.77(3H,d,J=0.8Hz),1.86(1H,dd,J=5.6,14.4Hz),2.30 (3H,S),2.36-2.44(4H,m),2.50-2.64(3H,m),2.66(1H,dd,J=2.4, 8.0Hz),2.89(1H,dt,J=2.4,6.0Hz),3.38-3.70(5H,m),3.75-3.81 (1H,m), 4.93(1H,d,J=10.0Hz),5.06(1H,d,J=10.8Hz),5.57 (1H,dd J=10.0,15.2Hz),5.71(1H,dd,J=9.6,15.2Hz),5.87(1H,d,J=15.2Hz), 6.13(1H,d,J=10.8Hz),6.53(1H,dd,J=11.2,15.2Hz)
ESI-MS m/z 637(M+H)⁺.

### Example 7

### Synthesis of (8E,12E,14E)-3,6,7,16,21-pentahydroxy-6,10,12,16,20-pentamethyl-18,19-epoxytricosa-8,12,14-trien-11-olide (Reference Example)

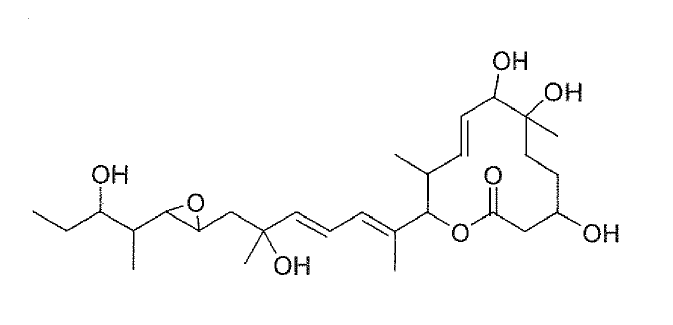

To a methanol solution (100.3 g) of (8E,12E,14E)-7-acetoxy-6,10,12,16,20-pentamethyl-3,6,16,21-tetrahydroxy-18,19-epoxytricosa-8,12,14-trien-11-olide (30.0g), methanol (306ml) was added. The mixture was stirred, to which potassium carbonate (0.72g, 5.21 mmol) was added. The mixture was stirred under room temperature for 4.5 hours. The reaction mixture was diluted with ethyl acetate (1529 ml), which was washed twice with 25% salt water (917 ml). The obtained organic layer was dried over sodium sulfate, filtrated and then concentrated under reduced pressure. To the concentrated product, acetonitrile (305 ml) was added and concentrated again under reduced pressure, to the concentrated product acetonitrile (30 ml) and tetrahydrofuran (30 ml) were added to give a solution (90.5 g, content 25.1 g) of the crudely purified subject compound.

### Example 8

### Synthesis of (8E,12E,14E)-7-((4-cycloheptylpiperazin-l-yl)carbonyl)oxy-6,10,12,16,20-pentamethyl-3,6,16,21-tetrahydroxy-18,19-epoxytricosa-8,12,14-trien-11-olide (Example)

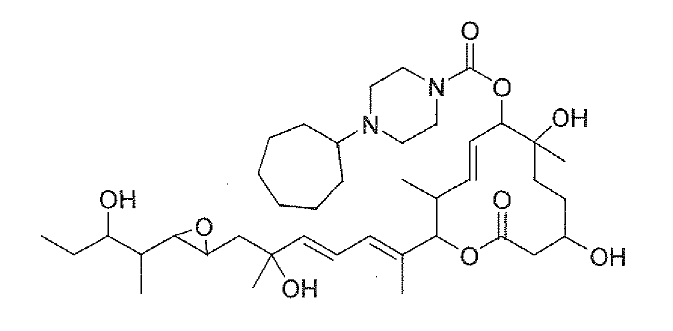

To crudely purified (8E,12E,14E)-3,6,7,16,21-pentahydroxy-6,10,12,16,20-pentamethyl-18,19-epoxytricosa-8,12,14-trien-11-olide (25.1 g, 49.2 mmol) in a mixed liquid of tetrahydrofuran (258 ml) and toluene (203 ml), dioctyl tin (IV) oxide (13.18 g, 36.5 mmol) was added, and the solution concentrated with heating to 100°C for 30 minutes with stirring. Further, after cooling it to 40°C, tetrahydrofuran (430 ml), 4-dimethylaminopyridine (5.5 g, 44.9 mmol) and 4-cycloheptylpiperazine carbonyl chloride (27.5 g, 112 mmol) were added. The mixture was stirred at 40°C for 3 hours. To the reaction mixture, toluene (287 ml) and tetrahydrofuran (287 ml) were added, the mixture was washed with a 5% ammonium chloride aqueous solution (573 ml) twice and with 25% salt water (573 ml). The obtained organic layer was dried over sodium sulfate, filtrated and then concentrated under reduced pressure to give a toluene solution (278 g, content 27.7 g) of the crudely purified subject compound.

### Example 9

### Purification of (8E, 12E, 14E) -7-((4-cycloheptylpiperazin-l-yl)carbonyl)oxy-6,10,12,16,20-pentamethyl-3,6,16,21-tetrahydroxy-18,19-epoxytricosa-8,12,14-trien-11-olide (Example)

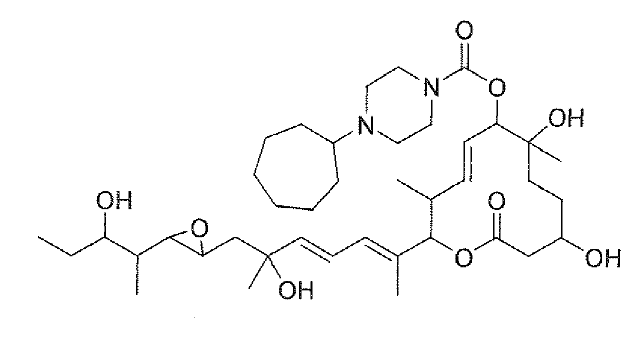

To the toluene solution (278 g) of the crudely purified (8E,12E,14E)-7-((4-cycloheptylpiperazin-1-yl)carbonyl)oxy-6,10,12,16,20-pentamethyl-3,6,16,21-tetrahydroxy-18,19-epoxytricosa-8,12,14-trien-11-clide (27.7 g) obtained in Example 8, methanol (420 ml) was added. The mixture was stirred at room temperature for 30 minutes, to which methanol (580 ml) was dropped under -10°C and the mixture was stirred for additional 1 hour at that temperature. The obtained suspension was filtrated by using a filtration auxiliary agent (Roka Help, Mitsui Mining And Smelting Company, Limited). The resultant was then subjected to washing with methanol (138 ml) to give a methanol solution (1500 ml) of (8E,12E,14E)-7-((4-cycloheptylpiperazin-1-yl)carbonyl)oxy-6,10,12,16,20-pentamethyl-3,6,16,21-tetrahydroxy-18,19-epoxytricosa-8,12,14-trien-11-olide (28.3 g). The obtained methanol solution (500 ml) of the crude product (10.0 g) of the subject compound was filtrated with an activated charcoal filter (ZetaCarbon, 90 mmφ, CUNO). The resultant was then subjected to washing with methanol (1000 ml). The filtrate and washing solution were mixed together and concentrated under reduced pressure to give a crude product of the subject compound (content 9.6 g). By the same operation, the remaining methanol solutions of the crude product were treated (total three batches, content 27.6 g). The obtained crude product of the subject compound was diluted with ethyl acetate (94 ml) and was filtrated by using silica gel (139 g, Chromatorex NH-DM2035, FUJI SILYSIA CHEMICAL LTD.) and then subjected to washing with methanol:ethyl acetate = 3:97 (2770 ml). The filtrate and washing solution were mixed together and concentrated under reduced pressure to give the crude product of the subject compound 42.9 g (content 27.4 g). To the obtained crude product of the subject compound (content 8.2 g), chloroform (58 ml) was added. The mixture was purified by using silica gel column chromatography (Chromatorex NH-DM2035, FUJI SILYSIA CHEMICAL LTD., elution solvents were 1) ethyl acetate:heptane = 70:30, 2) ethyl acetate:heptane = 85:15, 3) ethyl acetate, 4) methanol:ethyl acetate = 3:97, in this order). The purified fraction was collected and concentrated under reduced pressure to give (8E,12E,14E)-7-((4-cycloheptylpiperazin-l-yl)-carbonyl)oxy-6,10,12,16,20-pentamethyl-3,6,16,21-tetrahydroxy-18,19-epoxytricosa-8,12,14-trien-11-olide (content 7.9 g).
¹-NMRSpectrum(CD₃OD,400MHz)δ(ppm):0.89(3H,d,J=6.8Hz),0.90 (3H,d,J=6.8Hz),0.94(3H,t,J=7.2Hz),1.10-1.77(24H,m), 1.77 (3H,brs),1.79-1.90(3H,m),2.42-2.74(9H,m),2.85-2.92(1H,m), 3.36-3.70(5H,m),3.72-3.84(1H,m), 4.92(1H,dd,J=9.6,15.2Hz), 5.71(1H,dd,J=9.0,15.2Hz), 5.87(1H,d,J=15.2Hz), 6.13(1H,d,J=11.2Hz),6.52(1H,dd,J=11.2,15.2Hz)
ESI-MS m/z 719(M+H)⁺

## Claims

1. A method for producing a macrolide derivative represented by Formula (5) 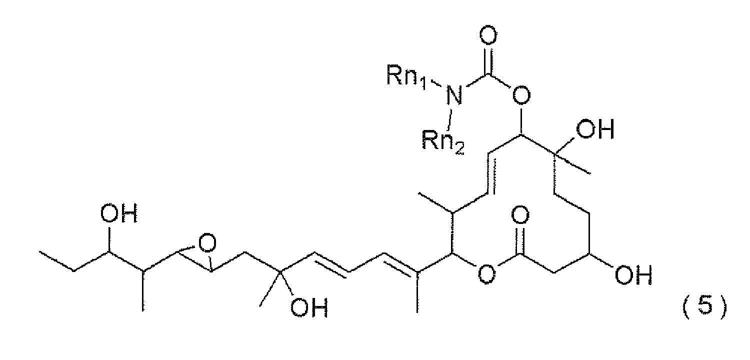 (where Rn₁ and Rn₂ may be the same as or different from each other, and represent
a) a hydrogen atom,
b) a C1 to C22 alkyl group that may have substituent(s),
c) an unsaturated C2 to C22 alkyl group that may have substituent(s),
d) a C6 to C14 aryl group that may have substituent(s),
e) a 5- to 14-membered ring heteroaryl group that may have substituent(s),
f) a C7 to C22 aralkyl group that may have substituent(s),
g) a 5- to 14-membered ring heteroaralkyl group that may have substituent(s),
h) a C3 to C14 cycloalkyl group that may have substituent(s),
i) a 3- to 14-membered non-aromatic heterocyclic group that may have substituent(s), or
j) a 3- to 14-membered non-aromatic heterocyclic group formed by Rn₁ and Rn₂ with a nitrogen atom that is bonded together (the 3- to 14-membered non-aromatic heterocyclic group may have substituent(s))),
comprising a step of reacting a tin (IV)-containing macrolide derivative represented by Formula (3) 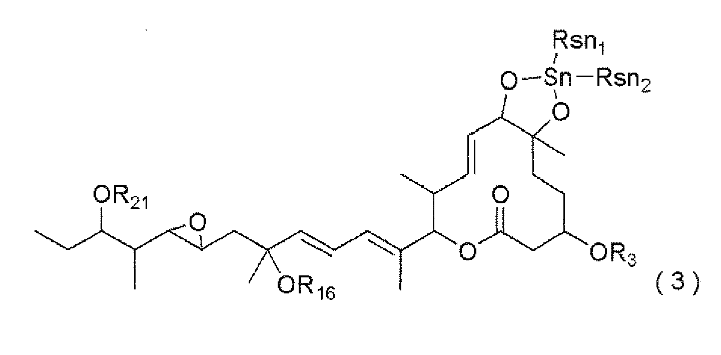 (where each of R₃, R₁₆ and R₂₁ independently represents a hydrogen atom or a protective group of hydroxyl group, and each of Rsn₁ and Rsn₂ independently represents a C1 to C30 alkyl group), in a solvent with a carbamoyl halide derivative represented by Formula (4) 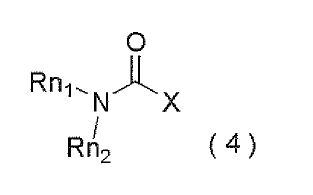 (where Rn₁ and Rn₂ are defined in the same way as above, and
X represents a halogen atom),
and, subsequently where necessary, subjecting the product to an elimination reaction of a protective group of hydroxyl group.

2. The production method according to Claim 1, further comprising a step of reacting a macrolide derivative represented by Formula (1) 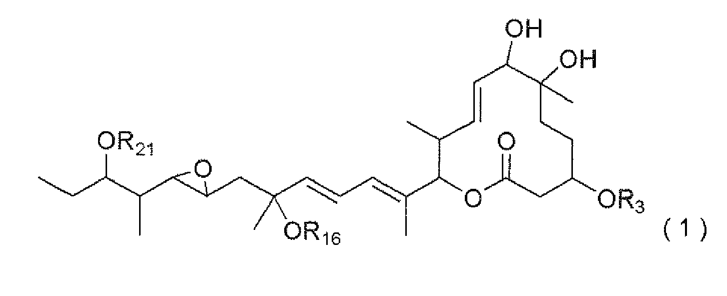 (where each of R₃, R₁₆ and R₂₁ independently represents a hydrogen atom or a protective group of hydroxyl group),
in a solvent with a diallyl tin (IV) oxide represented by Formula (2) 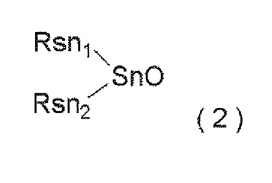 (where each of Rsn₁ and Rsn₂ independently represents a C1 to C30 alkyl group),
before reacting the tin (IV)-containing macrolide derivative represented by Formula (3) with the carbamoyl halide derivative represented by Formula (4).

3. The production method according to Claim 1 or 2, wherein the carbamoyl halide derivative represented by Formula (4) is a derivative represented by Formula (4-1) 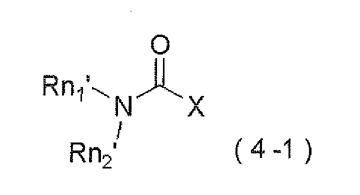 (where Rn₁' and Rn₂' form a 3- to 14-membered non-aromatic heterocyclic group with a nitrogen atom bonded together, (the 3- to 14-membered non-aromatic heterocyclic group may have substituent(s)), and X represents a halogen atom).

4. The production method according to Claim 1 or 2, wherein the carbamoyl halide derivative represented by Formula (4) is a derivative represented by Formula (4-2).

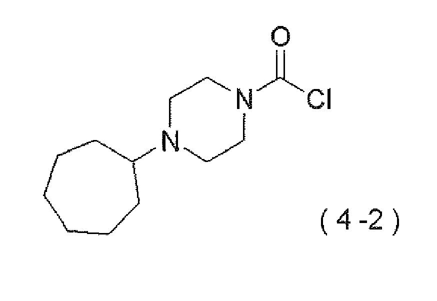

5. A method for producing a tin (IV) -containing macrolide derivative represented by Formula (3) 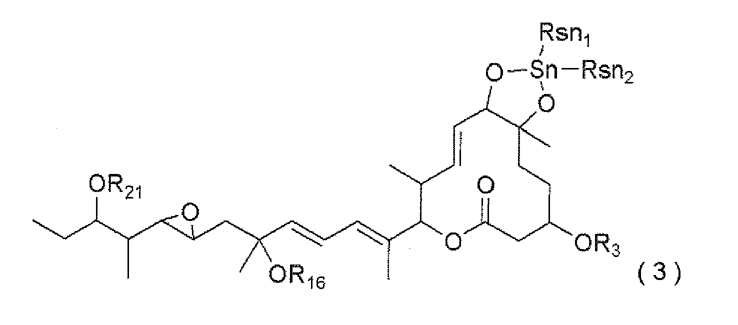 (where each of R₃, R₁₆ and R₂₁ independently represents a hydrogen atom or a protective group of hydroxyl group, and each of Rsn₁ and Rsn₂ independently represents a C1 to C30 alkyl group), comprising a step of reacting a macrolide derivative represented by Formula (1) 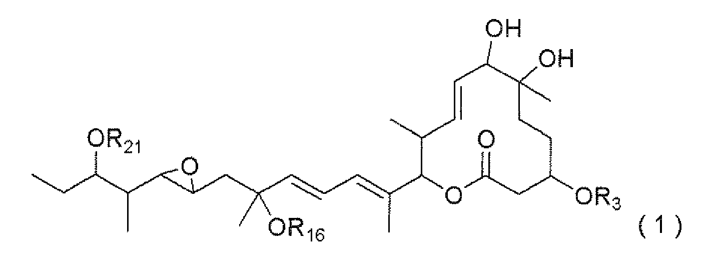 (where each of R₃, R₁₆ and R₂₁ independently represents a hydrogen atom or a protective group of hydroxyl group),
in a solvent with a dialkyl tin (IV) oxide represented by Formula (2) 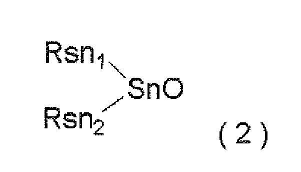 (where each of Rsn₁, and Rsn₂ independently represents a C1 to C30 alkyl group).

6. A tin (IV)-containing macrolide derivative represented by Formula (3) 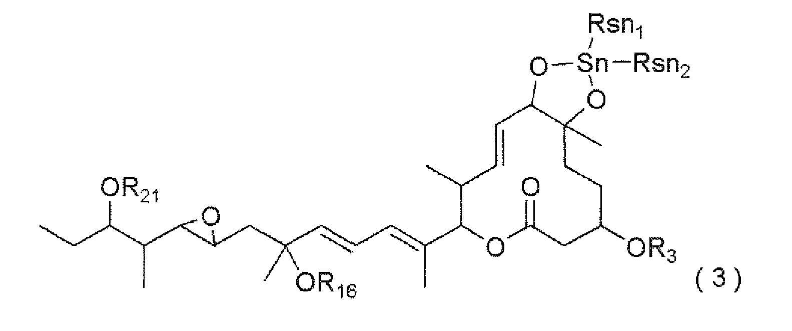 (where each of R₃, R₁₆ and R₂₁ independently represents a hydrogen atom or a protective group of hydroxyl group, and each of Rsn₁ and Rsn₂ independently represents a C1 to C30 alkyl group).

7. The tin (IV)-containing macrolide derivative according to claim 6, wherein R₃, R₁₆ and R₂₁ are hydrogen atoms.

8. The tin (IV)-containing macrolide derivative according to claim 6, wherein each of Rsn₁ and Rsn₂ independently represents a C1 to C10 alkyl group.

9. A carbamoyl halide derivative represented by Formula (4-2).
